# EUROPEAN PATENT SPECIFICATION

(11) **EP 0 656 759 B1**
(45) Date of publication and mention of the grant of the patent: **02.09.1998**
(21) Application number: 95904337.3
(22) Date of filing: 17.06.1994
(51) Int. Cl.: A61B 3/12, A61B 3/135

(54) **OCULAR FLUOROMETER**
FLUORESZENZMESSER FÜR AUGENUNTERSUCHUNGEN
FLUORIMETRE OCULAIRE

(30) Priority: 18.06.1993 PT 10129093
(43) Date of publication of application: 14.06.1995
(73) Proprietor: FERNANDES DA CUNHA VAZ, Jose Guilherme, P-3000 Coimbra (PT); PIRES DOMINGUES, Jose Paulo, 3000 Coimbra (PT); BOLOTA ALEXANDRE CORREIA, Carlos Manuel, P-3000 Coimbra (PT)
(72) Inventor: FERNANDES DA CUNHA VAZ, Jose Guilherme, P-3000 Coimbra (PT); PIRES DOMINGUES, Jose Paulo, 3000 Coimbra (PT); BOLOTA ALEXANDRE CORREIA, Carlos Manuel, P-3000 Coimbra (PT)
(74) Representative: Pereira da Cruz, Joao
(86) International application number: PCT/PT94/00005
(87) International publication number: WO 95/00068

(56) References cited:
- EP-A- 0 512 518
- WO-A-93/05699
- WO-A-93/20743
- DE-A- 3 117 699
- ARCHIVES OF OPHTHALMOLOGY, vol.101, no.11, November 1983 pages 1753 - 1756 R.C. ZEIMER ET AL. 'Vitreous Fluorophotometry for Clinical Research' cited in the application
- INVESTIGATIVE OPHTHALMOLOGY & VISUAL SCIENCE, vol.29, no.8, August 1988 pages 1285 - 1293 J.W. MC LAREN ET AL. 'A Scanning Ocular Spectrofluorophotometer' cited in the application

## Description

This invention is a new ocular fluorometer.

The instrument is adapted to an ophthalmic slit-lamp (1) and includes holders for fluorescence excitation and barrier filters (3,5) in the light path and a detector system made of a linear array of photodiodes (8) with respective circuitry for reading and control (9). The light is focused in a chosen zone of the eye (13) inducing fluorescence by using specific wavelengths. The fluorescence is measured by the detector (8) coupled to one of the eyepieces of the slit-lamp (1) or to a beam-splitter (7) placed before the eyepieces.

There is a system for digitisation of the signal from the detector which is connected to an IBM-compatible computer (11) where the data is stored and analysed.

The instrument has a spatial resolution of 50 mm (corresponding to the spacing between the photodiodes) and a sensitivity (lower level of detection) equivalent to the fluorescence of a fluorescein solution of 1 ng/ml. The excellent reproducibility and linearity of the instrument and its design are particularly appropriate for performing the most sophisticated ocular fluorometry measurements in the clinical ophthalmologic practice, including studies of the permeability of the blood-retinal barrier.

The present invention relates to a new ocular fluorometer. The instrument is based on an ophthalmic slit-lamp using specific pairs of fluorescence excitation and barrier filters in the light path and a detector system made by a photodiode linear array with respective circuitry for reading and control. The incoming light from the slit is focused in the eye location, where measurement of the induced fluorescence is desired. This in turn is measured using a detector system placed in one of the eyepieces of the slit-lamp microscope or using a beam-splitter, the other eyepiece being used for focusing. There is also a system for digitisation of the signal from the detector which is connected to a IBM compatible computer where the data is stored and analysed.

### DESCRIPTION OF PRIOR ART

Since 1975, when the technique of vitreous fluorophotometry was proposed for the first time, as a method designed to measure low concentrations of fluorescein in the eye, and more specifically in the vitreous body, various different ocular fluorometers have been proposed.

The ocular fluorometers presently available have serious limitations. Among them:
- instrument used optically for posterior segment examination;
- fixed angle of measurement;
- automatic scanning of the whole axis of the eye;
- fixed filter system used only for fluorescein detection.

This equipment is described in detail in: *Early Breakdown of the Blood-Retinal Barrier in Diabetes*, José G. Cunha-Vaz, J.R. Faria de Abreu, António J. Campos and Gabriela M. Figo, *British Journal of Ophthalmology*, volume 59, n° 11, pages 649-656, November 1975; *Vitreous Fluorophotometry for Clinical Research*, Ran C. Zeimer, Norman P. Blair. José G. Cunha-Vax, Arch.; patent DE 31 17 699 A1, Vassiliadis et al, Metricon, Ltd, 94043 Mountain View, Calif., US. Ophthalmol, vol. 101, pages 1753-1756, November 1983; *A Scanning Ocular Spectrofluorophotometer*, Jay W. Mclaren and Richard F. Brubaker, *Investigative Ophthalmology & Visual Science*, vol., 29, n° 8, pages 1285-1292, August 1988. **DA A3117699, Vassiliadis et al, - discloses an Ocular Fluorometer according to the preamble of claim 1**

### SUMMARY OF THE INVENTION

The fluorometer here described solves the technical problems mentioned previously and minimises production costs. It is an instrument adapted to an ophthalmic slit-lamp (a biomicroscope using slit illumination), where excitation and barrier filters have been placed in the light path and a detector based on an array of photodiodes with respective circuitry for reading and control. The illumination slit is focused in the eye location where the induced fluorescence is to be measured. This measurement is performed by a detector placed in one of the eyepieces of the slit-lamp; the other eyepiece is used for focusing. The equipment also includes a system for digitisation of the signal from the detector, which is connected to an IBM compatible computer where the data is stored and analysed.

One of the objectives of the invention is the conception of an instrument that is an accessory for adaptation to an ordinary ophthalmic slit-lamp; it is compact and more flexible for examination in various locations in the eye and not dedicated for posterior (rear) segment analysis. **The objective is achieved with the ocular fluorometer according to claim 1.**

This ocular fluorometer offers the possibility of using different angles of measurement. The instrument proposed is based on an original concept: it has a system that allows for automatic or manual positioning in selected areas of the eye of major clinical interest, the cornea/aqueous, aqueous/lens and vitreous/retina interfaces, performing electronic scanning in each of these areas.

It is therefore possible to obtain with this new system improved resolution and more rapid positioning in the areas of clinical interest.

An advantage of the invention is the possibility of quickly changing filters, both excitation and barrier. Such a device allows for detection of both exogenous and endogenous fluorescence, i.e. autofluorescence of ocular tissues.

This instrument also offers, when compared to the ocular fluorometers available, excellent computational capabilities.

Finally, this invention involves production costs which are calculated to be at least four times lower than for the instruments available.

### BRIEF DESCRIPTION OF DRAWINGS

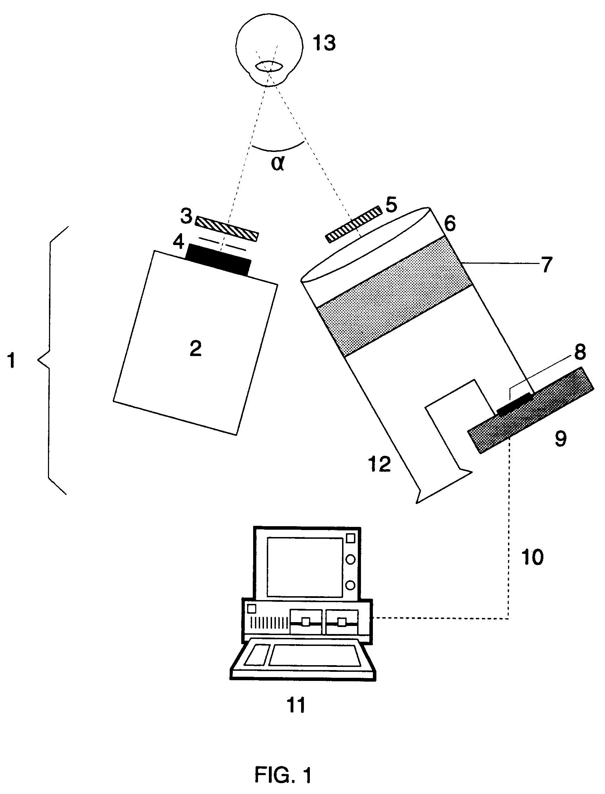

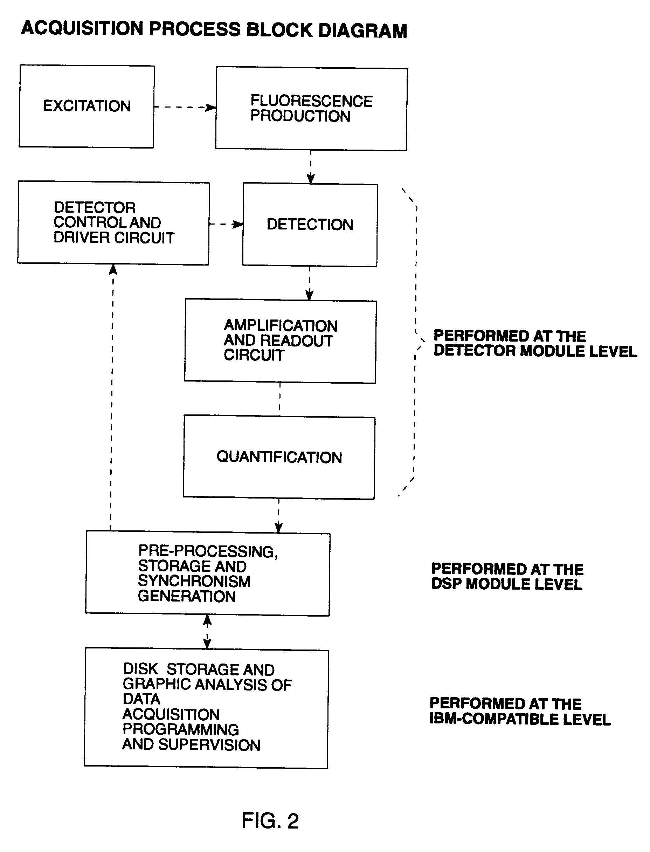

For information purposes and therefore without imposing undue restrictions, we include 2 drawings (figure 1 and figure 2):
- Figure 1 shows schematically the ocular fluorometer;
- Figure 2 shows the acquisition process block diagram;

### DETAILED DESCRIPTION OF A SELECTED SOLUTION

The invention is a fluorometric accessory adapted to an ophthalmic silt-lamp (biomicroscope with slit illumination), which is equipment that is present in every ophthalmic examination room.

The fluorometric accessory must be coupled and installed without difficulties, must allow the use of the slit-lamp when not in use for ocular fluorometry and must allow fluorometric measurements to be performed with high precision, reproducibility and liability, competing in its technical performance with the best existing ocular fluorometers available.

The conditions are therefore:
**1.** Easy installation of the unit, easy coupling and uncoupling to an ordinary ophthalmic slit-lamp without the need of a specialised technician.
**2.** To allow normal use of the slit-lamp when the fluorometric accessory is in place, without major hindrance.
**3.** To perform fluorometric measurements at the level of the cornea, anterior chamber, lens, vitreous and retina with the required resolution and reliability.
**4.** Easy interchange and placement of new filter pairs in order to be able to measure exogenous and endogenous fluorophores.
**5.** To perform fluorometric measurements in a reproducible manner with the sensitivity and linearity at least comparable to the instruments available on the market.

In order to fulfil these conditions the design of the instrument took into consideration the following:
**1.** Adaptation to the optical plane of the more distant eyepiece of the binocular system of the slit-lamp.
**2.** No interference with the optical system of the slit-lamp
**3.** Good resolution, even better than in presently available instruments.

A photodiode array (8) with a resolution between detectors of 50 mm was chosen. This corresponds "in vivo" to a resolution of 0.5 mm in the cornea-aqueous and aqueous-lens interfaces. This resolution is obtained using a measurement angle of 45°.

The instrument has the ability to measure at various angles (α, in figure 1), which opens up its fields of application for specular and light-scattering examination of the ocular structures. These different angles may also be kept constant if so desired, in order to maintain measurement reproducibility.

This instrument also performs separate and successive measurements on the cornea, lens and retina.

The existing alternatives for taking fluorometric measurements in different areas of the eye were based either on the capacity for mechanical automatic scanning along the axis of the eye or on isolated measurements in restricted areas of the eye (with the extent of the measurements depending on the resolution density of photodetectors).

While recognising the limitation associated both with the scanning of the entire eye, increasing the duration of the examination and resulting in a variety of artifacts and problems with eye fixation, and with the examination of only restricted areas of the eye, we have made an original option based on the average size of the human eye.

Three zones of measurement are chosen: cornea, lens and retina, the eye being approximately 24 mm long. This distance, as well as the location of the front surfaces of the cornea, lens and retina, may be determined experimentally using their specular reflection.

It was possible to develop a mechanical system for positioning by automatic control, using three main positions: Cornea (I), lens (II) and retina (III). After the initial focusing step on the front surface of the lens, the examiner only has to press positioning button I in order to focus on the cornea. By pressing button III it will bring into focus the detection system on the retinal region and, in this case, it is also necessary to change the instrumentation angle between incident light and collected light to 14° and to use a contact lens or a special 60D lens. These displacements are carried out using stepper motors under direct PC control.

Under these conditions it is possible to obtain fluorometric measurements characterised by very good resolution in the areas of principal clinical significance (cornea-aqueous, aqueous-lens and vitreous-retina interfaces).

The fluorescence peaks obtained in the recordings performed before administration of any exogenous fluorophore indicate the autofluorescence of the cornea, lens and retina and serve as references when reading the results.

The linear array of photodiodes can have two different orientations with a 90 degree difference. In the scanning position orientation, as discussed, the measurement gives the spatial distribution of fluorophore concentration along the region of interest of the ocular axis. In the spot position orientation, we lose the spatial resolution and all the photodiodes receive light from the same ocular region, the result being the integral of light from an eye location, this being particularly important for the front segment measurements.

Figure 1 shows the ocular fluorometer consisting essentially of a module for excitation (2), a module for detection (6,9), a module for reading and control , a processing module and a computer (11), which constitute the electronic component of the instrument.

The module of excitation (2) consists essentially of a slit-lamp with a device to receive appropriate excitation filters (3).

The instrument is coupled to a typical Zeiss-type slit-lamp by a simple adjustable-length tube-like mechanical device to provide image formation over the array of photodiodes. This is coupled to one port of the slit-lamp beam-splitter 7 and tightly fixed by the screwing ring of that port, or it is coupled by replacing one of the eyepieces and tightened by a similar method. In this latter case, because no auxiliary optics need to be added, we are limited to the optics of the slit-lamp. When the beam-splitter is used, auxiliary optics must be introduced in the light path inside the tube, in order to give some degree of freedom of focal length and image size.

The electronics of the instrument start at the detector chip (8) and ends at the computer (11), following the usual block diagram of any data acquisition and control systems, including:
1 - Transducer;
2 - Transducer readout circuit;
3 - Transducer driver and control circuit;
4 - Analogue-to-Digital conversion.
   For direct control of measurements and for data processing, the invention includes:
5 - A DSP-based PC processor module capable of running a local program, for data pre-processing, storage and direct control of measurements;
6 - A computer, running simultaneously a second program, for general supervision, disk storage and graphical analysis of results.

The photodetector is a Hamamatsu solid-state Self-Scanning Linear Photodiode Array, series S3921/S3924. It offers high performance and good flexibility and all the members of the series are pin-compatible. This kind of sensor features an integrated signal processing circuit providing an output signal with a boxcar waveform, thus allowing signal readout with a simple external circuit. Low dark current, large spectral range, good sensitivity and pixel pitch 50 µm (S3921) or 25 µm (S3924) are some other important characteristics (see Hamamatsu Mos Linear Image Sensors catalog for more technical details).

Driver/Readout circuit. The sensor driver and control circuit provides all the clock and control signals needed for the sensor to work properly (see technical data from Hamamatsu), with the timing parameters adjusted to the experiment requirements. The main pail of this circuit is the software programmable Intel 8254 Timer. The readout circuit includes signal conditioning, amplification and quantification by the ADC (BURR BROWN AD 7800). There is a clear interconection between driver and readout as the conversion timing of the ADC must be synchronized with sensor driving, determining the pulse output frequency and width.

The sensor exposure time is determined by the overall timing of the Driver/Readout circuit.

The PC Module is a DSP (Digital Signal Processing) board containing the Texas Instruments TMS320C25 digital signal processor specially developed for this Ocular Fluorometer. It is to be pluged into a free slot of an ordinary PC-compatible computer (11) and is connected to the detector module (9) (located at the ocular eye (12) end of the slit lamp (1)) by a 50-way flat cable (10). It has local memory accessed by both the TMS and the PC processor (11). It drives the sensor module and reads the converted data in a "stand alone" mode. It also performs some data preprocessing using some software noise reduction algorithms. In fact, the structure and speed of the DSP makes it possible to carry out arithmetic operations very quickly; this, in addition to ADC fast conversion time, allows us to make a large number of conversions per pulse and an average pulse amplitude that drastically reduces noise. Also, data can be converted into concentration units by the calibration function parameters.

An important element of this module is the aforementioned 8254 Timer, which is programmable by the PC but gated by the TMS and allows for acquisition timing maximum flexibility - one of the key features of the instrument.

The computer (11) is an interface between the user and the system. It gives the user the possibility of defining, in a friendly environment, some of the measurement parameters. After acquisition starts, the computer reads the pre-processed data and we can follow on the monitor in real time the acquisition result and decide whether the data is to be stored on disk for posterior analysis. We can also introduce some correction factors to compensate for eventual non-uniformities and/or non-linearities. Finally, the user can use the program for post-processing and to analyse data that has been stored on disk.

In figure 2 the acquisition process block diagram is presented, summarizing the functions of the instrument and stating which modules are responsible for them.

## Claims

1. An Ocular Fluorometer for measuring exogenous and endogenous fluorescence in specifically selected regions of clinical interest in the eye (13), such as the cornea/aqueous, aqueous/lens and vitreous/retina interfaces, being adapted to an ophthalmic slit-lamp (1) but allowing its simultaneous normal use, including filters for excitation and barrier functions (3,5) and further comprising an electronic component including a multiple photodiode detector (8), reading and control logic (9), and a computer (11) for general supervision, storage and graphic analysis of photometric results, characterised in that it includes a mechanical device which allows for automated positioning in three main positions of focusing, centred respectively on the anterior surface of the cornea, anterior surface of the lens and retina-vitreous interface, whereby this automated positioning is initiated with manual focusing, by an examiner, of the slit-lamp on the anterior surface of the lens, the examiner, after this initial step, having only to press a cornea positioning button (I) or a retina positioning button (III) of the mechanical device to bring automatically into focus, respectively, the area of the cornea or the vitreous-retina interface, whereby, when measurements are performed in the latter area, the angle of the instrument - between incident light and collected light - is changed to 14°, the pupil must be dilated and an accessory lens must be introduced into the path of light.

2. The Ocular Fluorometer according to claim 1 wherein the anterior surfaces of the cornea, lens and retina are identified by specular reflection.

3. The Ocular Fluorometer according to claim 1 further comprising a linear array of photodiodes (8), respective driver and readout circuits (9) for providing clock and control signals with timing parameters adjusted to the experiment requirements and for signal conditioning, amplification and quantification and controlled by a Digital Signal Processing (DSP) that maximises the Analog to Digital Conversion (ADC) data rate in order to drastically reduce noise, whereby the photodiodes detect the fluorescent light from an area in focus, allowing high resolution fluorescence measurements.

4. The Ocular Fluorometer according to claim 3, wherein said photodiode array (8), further comprising an ultimate limit in spatial resolution of 50µm corresponding to the pixel-to-pixel spacing and a Lower Level of Detection of 1 ng/ml fluorescein equivalent concentration.

5. The Ocular Fluorometer according to claim 3, further comprising a PC module based on a Digital Signal Processor and having local memory, plugged into an IBM compatible computer (11) slot, that drives the detector module (9) of said Ocular Fluorometer through a 50-way flat cable (10), reads converted data, performs some data pre-processing using software noise reduction algorithms and assures an efficient connection to the host computer (11) for measurements supervision, data storage and analysis, and graphical interface with the user.

6. The Ocular Fluorometer according to claim 5, further comprising two programs running simultaneously, one at the host computing means level and the other at the local processing means.

7. The Ocular Fluorometer according to claim 3, wherein said photodiode array is rotatable so as to act as both a spot and scanning fluorometer, a feature that is particularly important in the anterior chamber.

## Patentansprüche

1. Ein Augenfluorometer um exogene und endogene Fluoreszenz zu messen in spezifisch ausgewählten Augenregionen (13), mit klinischem Interesse, wie Hornhaut/Kammerwasser, Kammerwasser/Linse und Glaskörper/Netzhaut "interfaces", welches zu einer Augenschlitzlampe (1) angepasst ist aber sein simultanes normales Gebrauch erlaubt, und welches Filter für Erregung- und Sperrefunktionen (3,5) und auch ein elektronisches Bauelement einschliesst sowie einen mehrfachen Photodiodendetektor (8), Lesen und Kontrol-Steuerlogik (9) und einen Computer (11) für die allgemeine Überwachung, Speicherung und Graphikanalyse von photometrischen Resultaten aufweist, dadurch gekennzeichnet dass es eine mechanische Einheit umfasst, die die automatisierte Positionierung in drei Haupteinstellungen erlaubt, beziehungsweise auf der vorderen Oberfläche der Hornhaut, der Linse and der Netzhaut-Glaskörpers "interface", wobei diese Positionierung mit der von einem Untersucher handbetätigten Einstellung der Augenschlitzlampe auf die vordere Oberfläche der Linse initiiert wird, indem der Untersucher nur einen Hornhaut-Positionsknopf (I) oder einen Netzhaut-Positionsknopf (III) zu betätigen hat um die Gegend der Hornhaut oder der Glaskörper/Netzhaut "interface" automatisch einzustellen, wobei, wenn Messungen in dieser letzten Gegend durchgeführt werden, der Winkel des Instruments - zwischen eifallendes und gesammeltes Licht - zu 14° verändert wird, die Pupille erweitert werden muss und eine zusätzliche Linse in den Lichtpfad eingeführt werden muss.

2. Das Augenfluorometer nach Anspruch 1, für das Kennzeichen der vorderen Oberflächen der Hornhaut, der Linse und der Netzhaut durch spekularer Reflexion.

3. Das Augenfluorometer nach Anspruch 1, dass eine lineare Reihe von Photodioden (8) und die jeweiligen Treiber und Lesestromkreise (9) für Lese- und Kontrolsteuersignal mit Zeitparameter aufweist, die zu der Experimentanforderungen angepasst und für Signalbeschränkung, Verstärkung und Quantifikation durch ein DSP kontrolliert sind, welches die analog - zu - digital Datenkonvertierung maximisiert um das Geräusch drastisch zu verringern, wobei die Photodioden das fluoreszierende Licht von einem Fokusbereich ermitteln, um hohe Zerlegungs Fluoreszenzmessungen zu erlauben.

4. Das Augenfluorometer nach Anspruch 3, dass eine lineare Reihe von Photodioden (8) und ferner eine entscheidende Begrenzung in räumlich Zerlegung von 50 µm, dem Pixel-zu-Pixel Abstand entsprechend und einer niedrigeren Abfragungstufe von 1 ng/ml gleichwertigen Fluoreszeinkonzentration aufweist.

5. Das Augenfluorometer nach Anspruch 3, dass einen PC Modul aufweist, welches auf einen Digitalsignal Prozessor begründet und einen lokalen Speicher aufweisenden, in einem IBM kompatiblen Computer (11) eingeschaltet ist, der den Detektormodul (9) durch ein 50-way flaches Kabel (10) antreibt, die umwandelte Daten liest, einige Daten-aufbereitung macht durch Software Geräusch-verkleinerung Algorithmus, und einen leistungfähigen Anschluss zu dem Hauptrechner Computer (11) versichert, für die Messungenüberwachung, Daten-Speicherung und Analyse, durch graphischer "interface" mit dem Benutzer.

6. Das Augenfluorometer nach Anspruch 5, dass ferner zwei gleichzeitig laufende Programme aufweist, eines an den rechnenden Mitteln des Hauptrechners und das andere an den lokalen Verarbeitungsmitteln.

7. Das Augenfluorometer nach Anspruch 3, worin die erwähnte Photodiode-Reihe drehbar ist um als ein Punkt- und Abtastungfluorometer zu wirken, ein Merkmal dass in der vorderen Kammer besonders wichtig ist.

## Revendications

1. Un Fluoromètre Oculaire pour mesurer la fluorescence exogène et endogène dans des zones de l'oeil (13) spécifiquement sélectionnées en raison de leur intérêt clinique, comme les interfaces cornée / humeur aqueuse, humeur aqueuse / cristallin et humeur vitrée / rétine, adapté à une lampe à fente ophtalmologique (1) mais permettant l'usage normal des deux appareils simultanément , équipé de filtres d'excitation et de barrière (3, 5), d'un composant électronique comprenant un détecteur à photodiodes (8), des systèmes logiques de lecture et de contrôle et un ordinateur (11) pour la supervision générale du système, l'enregistrement des données et l'analyse graphique des résultats photométriques. Ce fluoromètre oculaire est caractérisé par un dispositif mécanique qui permet le positionnement automatique du point focal dans les trois positions principales suivantes - surface antérieure de la cornée, surface antérieure du cristallin et interface antérieure rétine/humeur vitrée, de façon que ce positionnement automatique soit initié par la focalisation manuelle de la lampe à fente sur la surface antérieure du cristallin, par un utilisateur qui n'aura qu'à appuyer sur un des boutons de positionnement (I) ou (II) du dispositif mécanique pour obtenir la focalisation automatique, respectivement de la cornée ou de l'interface humeur vitrée/rétine, étant nécessaire quand les mesures sont prises dans cette interface que l'angle de l'instrument - défini par la lumière incidente et la lumière reçue - soit fixé a 14°,que la pupille soit dilatée et que des éléments optiques accessoires - des lentilles- soient introduits dans le parcours du faisceau lumineux.

2. Le Fluoromètre Oculaire dans lequel, en accord avec la révendication n° 1, les surfaces antérieures de la cornée, du cristallin et de la rétine sont identifiées par réflexion speculaire.

3. Le Fluoromètre Oculaire équipé, en accord avec la révendication n° 1, d'un assemblage linéaire de photodiodes (8) avec les circuits respectifs de gestion et de lecture de signal (9) destinés à fournir, des signaux de temporisation et de contrôle, les paramètres de temporisation étant adaptables aux contraintes des mesures à exécuter, et au conditionnement, à l'amplification et à la quantification du signal de sortie, qui est contrôlé par un processeur digital du signal qui maximise la vitesse de la conversion analogique digitale de façon à réduire drastiquement le bruit, de façon à ce que les photodiodes détectent la lumière dans la zone focale, permettant l'obtention de mesures de fluorescence de haute résolution.

4. Le Fluoromètre Oculaire, en accord avec la révendication n° 3, où "ledit- assemblage" de photodiodes (8) possède une résolution spatiale de 50mm correspondant à l'écartement de pixel à pixel et une Limite Inférieure de Détection de 1 ng/ml concentration équivalente de fluorescéine.

5. Le Fluoromètre Oculaire comprenant, en accord avec la revendication n° 3, un module de digitalisation basé sur un microprocesseur de digitalisation de signal incorporé a un ordinateur personnel compatible IBM qui commande le module de détection "dudit- fluoromètre" oculaire à travers un câble plat de 50 conducteurs, lit les données converties, exécute un pré-traitement des données en utilisant un logiciel basé sur des algorithmes de réduction du bruit et assure une très bonne communication avec l'ordinateur pour la supervision des mesures, l'enregistrement et l'analyse des données et la fonction d'interface graphique avec l'utilisateur.

6. Le Fluoromètre Oculaire incluant, en accord avec la revendication n° 5, deux logiciels qui sont exécutés simultanément, l'un par les moyens de computation de l'ordinateur et l'autre par des moyens de computation spécifiques.

7. Le Fluoromètre Oculaire dans lequel, en accord avec la revendication n° 5, "ledit-assemblage" de photodiodes est orientable de façon à pouvoir fonctionner comme fluoromètre de tache et de balayage, cette caractéristique étant particulièrement importante dans l'étude de l'humeur aqueuse.
